# EUROPÄISCHE PATENTSCHRIFT

(11) **EP 2 184 040 B1**
(45) Veröffentlichungstag und Bekanntmachung des Hinweises auf die Patenterteilung: **15.05.2013**
(21) Anmeldenummer: 09174536.4
(22) Anmeldetag: 29.10.2009
(51) Int. Cl.: A61F 13/10

(54) **Epikondylitisspange**
Epicondylitis strap
Pince pour épicondylite

(30) Priorität: 05.11.2008 DE 102008055867
(43) Veröffentlichungstag der Anmeldung: 12.05.2010
(73) Patentinhaber: Bauerfeind AG, 07937 Zeulenroda (DE)
(72) Erfinder: Bätz, Ronny, 07937, Vogtländisches Oberland (DE); Stier, Gerald, 07937, Langenwetzendorf (DE)
(74) Vertreter: Bardehle, Heinz

(56) Entgegenhaltungen:
- US-A- 4 763 901
- US-A- 5 152 302
- US-A- 6 007 508

## Beschreibung

Die Erfindung bezieht sich auf eine Epikondylitisspange, deren den Arm umfassendes Fassungsteil durch ein stufenlos verstellbares Halteband zusammenziehbar ist und die an ihrer Innenseite zur Druckausübung auf eine Druck erforderliche Stelle eine Pelotte aufweist, die als längliches Polster an dem Fassungsteil befestigt ist. Eine derartige Spange ist in der DE 197 16 705 C1 beschrieben und dargestellt. Bei der Druck erfordernden Stelle kann es sich um den Epikondylus handeln, der beim sog. Tennisarm (Epicondylitis radialis humeri) die betreffende Stelle ist. Daneben besteht auch ein Bedürfnis im Falle des sog. Golferarmes (Epicondylitis ulnaris humeri) eine Stelle am Arm einem Druck auszusetzen, die am Arm entgegengesetzt zu dem Epikondylus liegt. Dabei handelt es sich um Beispiele für Anwendungsfälle von therapeutisch erforderlichen Lagen, die im Einzelfall vom behandelnden Arzt zu entscheiden sind. Um diese Spange sowohl für den rechten als auch für linken Arm anwendbar zu gestalten, ist die Pelotte mittels einer senkrecht zur Längsrichtung der Pelotte liegenden Achse drehbar an der Spange abnehmbar befestigt, so dass sie hinsichtlich ihrer länglichen Gestaltung jeweils in diejenige Lage gebracht werden kann, in der sie entweder am rechten oder linken Arm jeweils die Druckausübung auf den Epikondylus ausübt.

Die vorstehend beschriebene Epikondylitisspange muss über ihre Pelotte einen erheblichen Druck auf die betreffende Stelle des Armes ausüben, damit die gewollte therapeutische Wirkung eintritt, wobei sich beim Anlegen der Spange und dem Zusammenziehen des Haltebandes Schubkräfte auf die Achse der Pelotte ergeben, die diese erheblich belasten, was zu einer Beschädigung der Achse führen kann. Es hat sich außerdem herausgestellt, dass beim Anlegen unter Umständen die Spange am Arm entweder zu hoch, zu tief oder verdreht angelegt worden ist und danach vom Benutzer wieder verschoben werden muss, um die Spange mit ihrer Pelotte an die richtige Stelle zu bringen, was ebenfalls eine erhebliche Belastung für die Achse der Pelotte erbringt.

Der Erfindung liegt die Aufgabe zugrunde, das Anlegen der Epikondylitisspange für eine Vielzahl von Anwendungsfällen, insbesondere dem Tennisarm und dem Golferarm, jeweils an der therapeutisch richtigen Stelle, und zwar am rechten oder linken Arm zu erleichtern und die gefundene Lage auch bei wiederholtem Anlegen zu sichern. Erfindungsgemäß geschieht dies dadurch, dass an einer Seite des Fassungsteils gegenüberliegend der von dem Halteband überbrückbaren Öffnung des Fassungsteils diese einen Lappen aufweist, der bei angelegtem Fassungsteil das Ellbogengelenk abdeckt und durch eine Aufnahme des Ellbogens dieses so umfasst, dass beim Anlegen des Fassungsteils der von dem Ellbogen geführte Lappen als Positionierungshilfe das Fassungsteil positioniert, das sowohl am rechten als auch am linken Arm mittels der Positionierungshilfe anbringbar ist und für die Anbringung am rechten bzw. linken Arm jeweils auf ihrer Innenseite eine Aufnahme für die Pelotte aufweist, über die die Pelotte wahlweise abnehmbar an dem Fassungsteil durch Haltemittel derart befestigt ist, dass das Fassungsteil sowohl am rechten als auch am linken Arm in jeweils richtiger Lage der Pelotte zum Epikondylus sitzt.

Bei der Anbringung der Pelotte an zwei möglichen gegenüberliegenden Stellen der Spange je nachdem, ob diese am rechten oder linken Arm angelegt wird bzw. den Tennisarm oder den Golferarm behandeln soll, ergibt sich jeweils eine sichere Befestigung für die Pelotte, die nach ihrem Anbringen nicht irgendwie noch in besonderer Weise eingestellt werden muss. Dabei dient der aus der Spange herauswachsende Lappen als Positionierhilfe dafür, dass in jedem Falle die eine funktionelle Einheit mit dem Lappen bildende Epikondylitisspange an der richtigen Stelle angelegt wird und nachträglich nicht mehr verschoben werden muss, womit weiterhin die Sicherheit der Befestigung der Pelotte gewährleistet bleibt. Durch die Kombination von Positionierhilfe durch den Lappen und die wahlweise gestaltete Anbringungsmöglichkeit der Pelotte ist die erfindungsgemäße Epikondylitisspange umfassend für alle bekannten Anwendungsfälle von Beschwerden am Arm, insbesondere durch sportliche Tätigkeiten, erfasst, womit für alle diese Behandlungserfordernisse der behandelnde Arzt mit einer einzigen entsprechend anpassbaren Epikondylitisspange auskommt.

Zweckmäßig kann man zur Bildung der Aufnahme den Lappen mit einem Loch versehen, das beim Anlegen der Spange den Ellbogen aufnimmt. Es ist aber auch möglich, den Lappen mit einer Aushöhlung zu versehen, die dann als Aufnahme für den Ellbogen dient und diesen besonders schützt.

Eine zweckmäßige Befestigung der Pelotte kann durch einen die Haltemittel bildenden Klettverschluss bestehen. Es ist aber auch möglich, die Haltemittel als Raststifte und diese aufnehmenden Bohrungen zu bilden. Dabei können die Raststifte entweder an der Pelotte und die zugehörigen Bohrungen an der Spange ausgebildet sein, es ist aber auch möglich, die Raststifte an der Spange und die wiederaufnehmenden Bohrungen an der Pelotte auszubilden.

Um beim Anlegen der Epikondylitisspange mit Sicherheit die zu behandelnde Stelle zu treffen, gestaltet man die Pelotte in Umfangsrichtung des Fassungsteils zweckmäßig solange, dass sie sich über die Druck erfordernden Stellen hinaus erstreckt. Vorteilhaft geschieht dies durch eine Gestaltung, gemäß der die Pelotte aus mehreren nebeneinander angeordneten Einzelpolstern besteht.

Um das Tragen der Epikondylitisspange angenehm zu gestalten, bildet man das Fassungsteil zweckmäßig so aus, dass dieses gegenüber seinem inneren Bereich einen weicheren Rand besitzt.

In den Figuren ist ein Ausführungsbeispiel der Erfindung dargestellt. Es zeigen:
- Fig.1: die Epikondylitisspange mit geschlossenem Halteband und einen Lappen mit einem Loch als Aufnahme des Ellbogens;
- Fig. 2: die Epikondylitisspange gemäß Figur 1 mit und einem Lappen mit einer Aushöhlung zur Aufnahme des Ellbogens;
- Fig. 3: einen Schnitt längs der Linie III-III aus Figur 2;
- Fig. 4: die am gebeugten Arm angelegte Epikondylitisspange;
- Fig. 5: die Epikondylitisspange gemäß Figur 2 mit geöffnetem Halteband und einer bezüglich Figur 2 gegenüberliegend angebrachten Pelotte;
- Fig. 6: einen Schnitt längs der Linie VI-VI aus Figur 5.

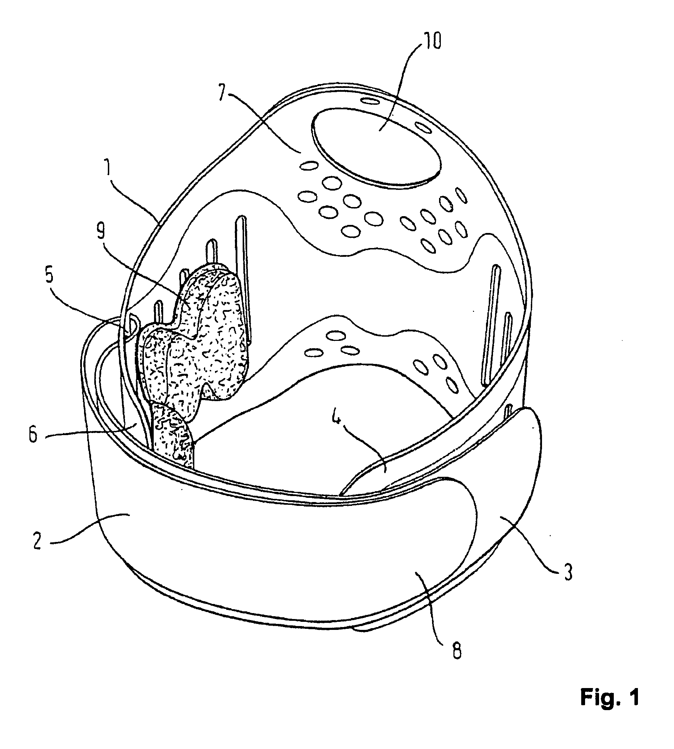

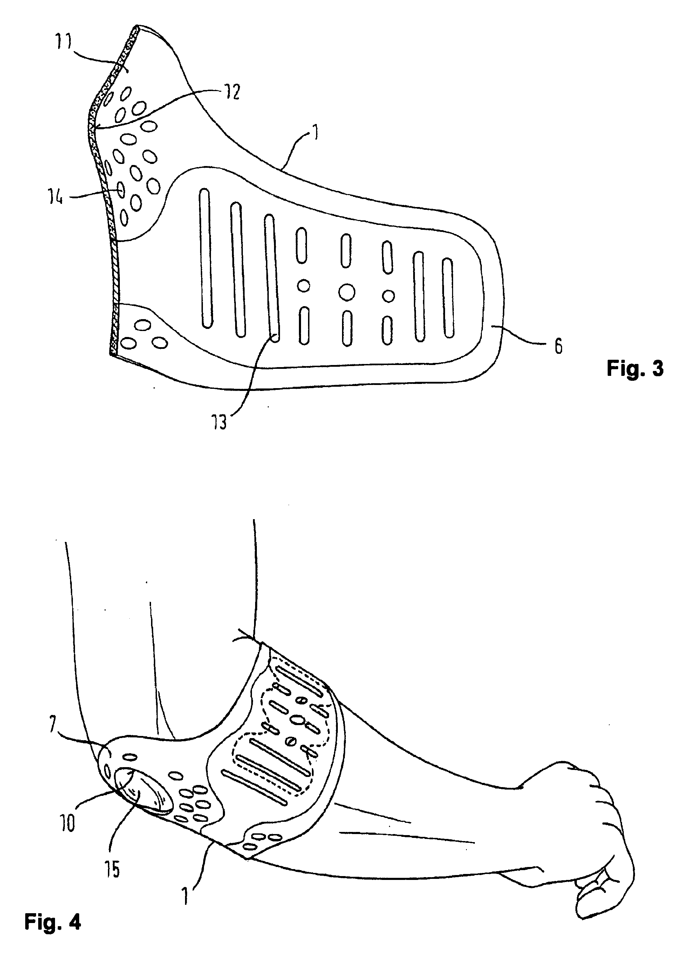

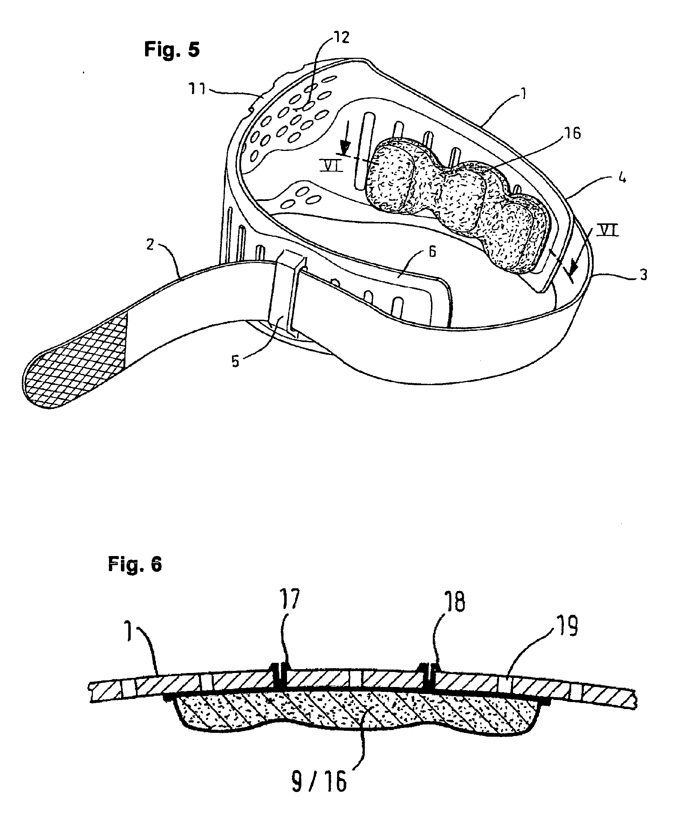

Die in der Figur 1 dargestellte Epikondylitisspange besteht aus dem Fassungsteil 1, das bei seiner Anlage am Arm (siehe Figur 4) diesen teilweise umfasst. Dabei lässt das Fassungsteil 1 zwischen seinen Enden 4 und 6 einen Zwischenraum frei, der von dem Halteband 2 überbrückt wird. Das Halteband 2 ist durch die längliche Öse 5 (siehe auch Figur 5) an dem einen Ende 6 des Fassungsteils 1 geschlungen, wobei sein äußerer Teil mit seinem einen Auslauf 8 an den anderen Auslauf 3 des Haltebandes 2 angelegt und an diesem mittels eines Klettverschlusses befestigt ist. Dieser Auslauf 3 ist ebenfalls mittels eines Klettverschlusses an dem Ende 4 des Fassungsteils 1 festgelegt. Diese Verbindung von Ende 4 und Auslauf 3 kann auch auf andere Weise vollzogen werden, beispielsweise durch in versetzte Löcher einrastende Stifte (siehe z.B. Figur 6). Durch entsprechend strammes Anziehen des Haltebandes 2 lässt sich das Fassungsteil 1 fest um einen Arm spannen. Diese Spannung des Haltebandes 2 überträgt sich somit auf das Fassungsteil 1 und drückt dieses fest an den Arm an. Bei dieser Art der Spannung der Epikondylitisspange handelt es sich um eine bekannte Handhabung.

Auf der den beiden Enden 4 und 6 des Fassungsteils 1 gegenüberliegenden Seite besitzt dieses einen Lappen 7, der eine Wölbung mit einem Loch 10 bildet, die bei Anlegen der Epikondylitisspange an einen Unterarm neben dem Armgelenk dieses von außen umfasst und mit dem Loch 10 den Lappen 7 und damit das Fassungsteil 1 zu dem Ellbogen in der therapeutisch erforderlichen Lage positioniert (siehe auch Figur 4).

Auf seiner einen Innenseite trägt das Fassungsteil 1 die Pelotte 9, die hier aus drei nebeneinander angeordneten zusammenhängenden Einzelpolstern besteht und die in bekannter Weise den gewünschten Druck auf die zu behandelnde Stelle des Arms ausübt. Wie in der Figur 1 dargestellt, kann die Epikondylitisspange sowohl am rechten als auch am linken Arm angelegt werden, wozu die Pelotte entsprechend auf der Innenseite des Fassungsteils 1 an gegenüberliegenden Stellen angebracht werden kann.

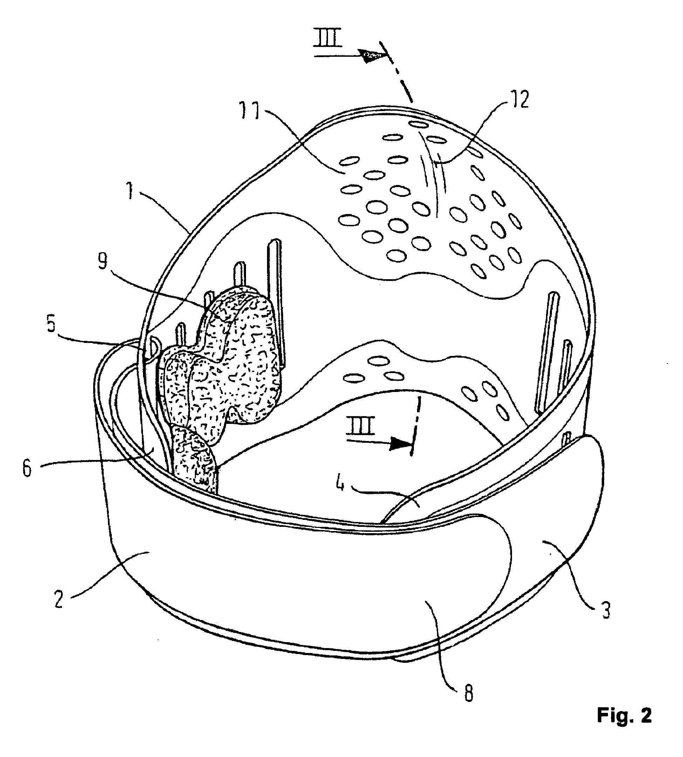

In der Figur 2 ist die Epikondylitisspange, die hinsichtlich ihres prinzipiellen Aufbaus demjenigen gemäß Figur 1 entspricht, mit einem anders gestalteten Lappen 11 dargestellt, der anstelle des Lochs 10 aus Figur 1 als Positionierhilfe mit einer Aushöhlung 12 versehen ist, die deutlich aus der Schnittzeichnung gemäß Figur 3 hervorgeht, die einen Schnitt längs der Linie III-III aus Figur 2 zeigt. Die Aushöhlung 12 stülpt sich schützend über den Ellbogen und fixiert damit die Epikondylitisspange gegenüber dem Ellbogengelenk in der therapeutisch notwendigen Position. Bezüglich der weiteren Gestaltungsteile der Epikondylitisspange gemäß Figur 2 sei auf die Erläuterungen zu Figur 1 verwiesen.

Figur 3 zeigt einen Schnitt durch das Fassungsteil 1 längs der Linie III-III aus Figur 2, in der deutlich die Aushöhlung 12 dargestellt ist, in die bei Anlegen der Epikondylitisspange der Ellbogen gewissermaßen hineinschlüpft und damit das Fassungsteil 1 zusammen mit der Epikondylitisspange richtig positioniert, Ergänzend sei noch darauf hingewiesen, dass das Fassungsteil 1 mit einer Reihe von Schlitzen 13 und Öffnungen 14 versehen ist, die dafür sorgen, dass bei angelegter Epikondylitisspange zur Haut des Armes in genügender Weise ein Luftzutritt ermöglicht wird.

Figur 4 zeigt die erfindungsgemäße Epikondylitisspange mit ihrem Fassungsteil 1 angelegt neben dem Ellbogengelenk eines Arms, wobei der Lappen 7 sich über den Ellbogen erstreckt. Das Loch 10 im Lappen 7 umfasst dabei den Ellbogen 15, der durch das Loch 10 hindurch tritt und damit die Lage der Epikondylitisspange fixiert.

In der Figur 5 ist die in der Figur 2 dargestellte Epikondylitisspange bei gelöstem Halteband 2, also bei geöffnetem Fassungsteil 1, dargestellt, wobei in Bezug auf die Darstellung gemäß Figur 2 die Pelotte 16 auf der Innenseite des Fassungsteils 1 an einer Stelle angebracht ist, die zu der Lage der Pelotte 9 gemäß Figur 2 entgegengesetzt ist. Die Pelotte 9 bzw. 16 ist hierzu in besonderer Weise mit dem Fassungsteil 1 jeweils verbunden, was nachstehend gemäß Darstellung in Figur 6 erläutert sei.

Die Anbringung der Pelotte 9 bzw. 16 an dem Fassungsteil 1 ist in der Figur 6 im Schnitt dargestellt, der längs der Linie VI-VI aus Figur 5 verläuft. Hieraus ergibt sich, dass an der Pelotte 9 bzw. 16 die beiden Raststifte 17 und 18 angebracht sind, die als bekannte geschlitzte Federstifte mit Widerhaken ausgebildet sind und jeweils eine Bohrung in dem Fassungsteil 1 durchsetzen. Freie Bohrungen sind mit dem Bezugszeichen 19 versehen. In der in der Figur 6 dargestellten Lage ist die Pelotte 9 bzw. 16 sicher am Fassungsteil 1 angebracht, kann jedoch von diesem dadurch wieder abgenommen werden, dass die Raststifte 17 und 18 zusammengedrückt werden, woraufhin diese durch die Bohrungen 19 herausgezogen werden können, womit die Pelotte 9 bzw. 16 von dem Fassungsteil 1 gelöst ist und gegebenenfalls auf der gegenüberliegenden Seite des Fassungsteils 1 in der gleichen Weise, wie in Figur 6 dargestellt, befestigt werden kann. Zu diesem Zweck besitzen also die beiden Enden 4 und 6 des Fassungsteils 1 jeweils mehrere entsprechende Bohrungen, so dass die Pelotte 9 bzw. 13 entweder auf der einen Seite oder auf der anderen Seite des Fassungsteils 1 angebracht werden kann, gegebenenfalls seitlich verschoben, wie dies in Gegenüberstellung die Figuren 2 und 3 auch zeigen.

Es sei noch darauf hingewiesen, dass anstelle der Raststifte 17 und 18 und der Bohrungen 19 die Pelotte 9 bzw. 16 mit einem Klettverschluss versehen werden kann, der gegen einen entsprechenden Belag auf der Innenseite des Fassungsteils 1 angedrückt werden kann, womit ebenfalls eine lösbare Verbindung zwischen Pelotte 9 bzw. 16 und Fassungsteil 1 herbeigeführt ist.

Durch diese wahlweise Anbringungsmöglichkeit der Pelotte 9/16 auf der einen oder gegenüberliegenden Seite des Fassungsteils 1 ist die erfindungsgemäße Epikondylitisspange sowohl für eine entsprechende Behandlung des rechten oder linken Arms oder beispielsweise des Tennisarms oder Golferarms geeignet, wobei in allen Fällen durch den Lappen 7 mit seinem Loch 10 oder seiner Aushöhlung 12 beim Anlegen der Epikondylitisspange diese durch den Ellbogen des Ellbogengelenkes richtig und jeweils in gleicher Weise automatisch positioniert wird, was die richtige Nutzung und Behandlung in einer Vielzahl von Behandlungsfällen durch die Epikondylitisspange erheblich erleichtert.

## Patentansprüche

1. Epikondylitisspange, deren den Arm umfassendes Fassungsteil (1) durch ein stufenlos verstellbares Halteband (2) zusammenziehbar ist und die an ihrer Innenseite zur Druckausübung auf eine Druck erforderliche Stelle eine Pelotte (9, 16) aufweist, die als längliches Polster an dem Fassungsteil (1) befestigt ist, **dadurch gekennzeichnet, dass** an einer Seite des Fassungsteils (1) gegenüberliegend der von dem Halteband (2) überbrückbaren Öffnung des Fassungsteils (1) diese einen Lappen (7, 11) aufweist, der bei am Arm angelegtem Fassungsteil (1) das Ellbogengelenk abdeckt und durch eine Aufnahme des Ellenbogens (5) dieses so umfasst, dass beim Anlegen des Fassungsteils (1) der von dem Ellbogen (5) geführte Lappen (7, 11) als Positionierungshilfe das Fassungsteil (1) positioniert, das sowohl am rechten als auch am linken Arm mittels der Positionierungshilfe anbringbar ist und für die Anbringung am rechten bzw. linken Arm jeweils an seiner Innenseite eine Aufnahme für die Pelotte (9, 16) aufweist, über die die Pelotte (9, 16) wahlweise abnehmbar an dem Fassungsteil (1) durch Haltemittel (17, 18, 19) derart befestigt ist, dass das Fassungsteil (1) sowohl am rechten als auch am linken Arm in jeweils der therapeutisch erforderlichen Lage der Pelotte sitzt.

2. Epikondylitisspange nach Anspruch 1, **dadurch gekennzeichnet, dass** der Lappen (7) mit einem Loch (10) als Aufnahme des Ellbogens (5) versehen ist.

3. Epikondylitisspange nach Anspruch 1, **dadurch gekennzeichnet, dass** der Lappen (7) mit einer Aushöhlung (8) als Aufnahme des Ellbogens (5) versehen ist.

4. Epikondylitisspange nach einem der Anspruch 1 bis 3, **dadurch gekennzeichnet, dass** die Haltemittel aus einem Klettverschluss bestehen.

5. Epikondylitisspange nach einem der Ansprüche 1 bis 3, **dadurch gekennzeichnet, dass** die Haltemittel aus Raststiften (17,18) und diese aufnehmenden Bohrungen (19) bestehen.

6. Epikondylitisspange nach einem der vorhergehenden Ansprüche, **dadurch gekennzeichnet, dass** die Pelotte (9, 16) in Umfangsrichtung des Fassungsteils (1) so lang ausgebildet ist, dass sie sich über die Druck erfordernde Stelle hinaus erstreckt.

7. Epikondylitisspange nach einem der vorhergehenden Ansprüche, **dadurch gekennzeichnet, dass** die Pelotte (1, 16) aus mehreren nebeneinander angeordneten Einzelpolstern besteht.

8. Epikondylitisspange nach einem der vorhergehenden Ansprüche, **dadurch gekennzeichnet, dass** das Fassungsteil (1) einen gegenüber seinem inneren Bereich weicheren Rand besitzt.

## Claims

1. An epicondylitis brace comprising a cuff (1) surrounding a patient's arm, which cuff can be tightened by means of a continuously adjustable support strap (2) and has a pad (9, 16) provided on its inner side for exerting pressure on a site that needs to be pressurized, said pad being attached to said cuff (1) in the form of elongate padding **characterized in that** provided on the side of the cuff (1) opposite the opening of said cuff (1) that can be bridged by said support strap (2) is a flap portion (7, 11) which will cover the elbow joint when the cuff (1) has been positioned round a patient's arm and encompass said elbow joint by accommodating the elbow (15) in such a way that when the cuff (1) is applied, the flap portion (7, 11) will be guided by the elbow (15) and thus function as a positioning aid for properly positioning said cuff (1), which cuff (1) can be disposed on either the right or the left arm of a patient by means of said positioning aid and includes receiving means for said pad (9, 16) on its inner side for mounting the brace on a patient's right or left arm, said pad receiving means allowing said pad (9, 16) to be optionally and removably attached to said cuff (1) by fastening means (17, 18, 19) in such a way that the cuff (1) will ultimately surround a patient's right or left arm with the pad (9, 16) in the proper therapeutically required position.

2. The epicondylitis brace of claim 1 **characterized in that** said flap portion (7) includes an opening (10) for accommodating a patient's elbow (15) therein.

3. The epicondylitis brace of claim 1 **characterized in that** said flap portion (7) includes a recess (8) for accommodating a patient's elbow (15) therein.

4. The epicondylitis brace of one of claims 1 to 3 **characterized in that** said fastening means consist of a hook and loop fastener.

5. The epicondylitis brace of one of claims 1 to 3 **characterized in that** said fastening means consist of locking pins (17, 18) and bores (19) accommodating said pins.

6. The epicondylitis brace of one of the preceding claims **characterized in that** the length of said pad (9, 16) in the circumferential direction of the cuff (1) has been chosen such that it extends beyond the site where pressure needs to be applied.

7. The epicondylitis brace of one of the preceding claims **characterized in that** said pad (9, 16) consists of plural single pads that are disposed adjacent to each another.

8. The epicondylitis brace of one of the preceding claims **characterized in that** said cuff (1) is softer at its edge than on the inside.

## Revendications

1. Bracelet épicondylien, dont la pièce de support (1) qui ceinture le bras peut être contractée de façon continue à l'aide d'une courroie de rétention (2), doté d'un coussinet (9, 16) sur sa face intérieure qui sert à exercer une pression sur un emplacement nécessitant une pression et qui est fixé à la pièce de support (1) tel un rembourrage oblong, **caractérisée en ce que**, sur une face de la pièce de support (1) située à l'opposé de l'ouverture de la pièce de support (1) qui peut être comblée par la courroie de rétention (2), elle présente une languette (7, 11) qui recouvre le coude lorsque la pièce de support (1) ceinture le bras et qui, dans sa prise du coude (5), entoure celui-ci de façon à ce que, lors de la pose de la pièce de support (1), la languette (7, 11) dirigée par le coude (5) agisse d'accessoire de positionnement de la pièce de support (1), qui peut être posée tant sur le bras droit que le gauche au moyen de l'accessoire de positionnement et qui, en vue d'être attachée tant au bras droit que gauche, présente sur chacune des faces intérieures respectives une prise pour les coussinets (9, 10), par laquelle le coussinet (9, 16) est fixé à la pièce de support (1) à l'aide d'attaches (17, 18, 19) de façon à pouvoir en être facultativement détaché et de façon à ce que la pièce de support (1) puisse être installée tant autour du bras droit que gauche, dans la position nécessaire à l'obtention des effets thérapeutiques.

2. Bracelet épicondylien selon la revendication 1, **caractérisé en ce que** la languette (7) est dotée d'un trou (10) en vue de la prise du coude (5).

3. Bracelet épicondylien selon la revendication 1, **caractérisé en ce que** la languette (7) est dotée d'une cavité (8) en vue de la prise du coude (5).

4. Bracelet épicondylien selon l'une des revendications 1 à 3, **caractérisé en ce que** les attaches sont composés de fermetures en velcro.

5. Bracelet épicondylien selon l'une des revendications 1 à 3, **caractérisé en ce que** les attaches sont composés de goupilles d'indexation (17, 18) et de percées (19) servant à accueillir ces dernières.

6. Bracelet épicondylien selon l'une des revendications précédentes, **caractérisé en ce que** le coussinet (9, 16) est formé en périphérie de la pièce de support (1) de façon si allongée, qu'elle s'étend sur l'entièreté de la surface nécessitant une pression.

7. Bracelet épicondylien selon l'une des revendications précédentes, **caractérisé en ce que** le coussinet (1, 16) est composé de plusieurs rembourrages individuels positionnés les uns à côté des autres.

8. Bracelet épicondylien selon l'une des revendications précédentes, **caractérisé en ce que** la pièce de support (1) dispose d'un rebord souple vis-à-vis de son espace intérieur.
